# EUROPEAN PATENT SPECIFICATION

(11) **EP 4 274 469 B1**
(45) Date of publication and mention of the grant of the patent: **13.08.2025**
(21) Application number: 22700875.2
(22) Date of filing: 06.01.2022
(51) Int. Cl.: A61B 3/06, A61F 2/16

(54) **LENSES, SYSTEMS, AND METHODS FOR REDUCING NEGATIVE DYSPHOTOPSIA**
LINSEN, SYSTEME UND VERFAHREN ZUR REDUZIERUNG VON NEGATIVER DYSPHOTOPSIE
LENTILLES, SYSTÈMES ET PROCÉDÉS DE RÉDUCTION DE LA DYSPHOTOPSIE NÉGATIVE

(30) Priority: 07.01.2021 US 202163134946 P
(43) Date of publication of application: 15.11.2023
(73) Proprietor: Amo Groningen B.V., 9728 NX Groningen (NL)
(72) Inventor: ALARCON HEREDIA, Aixa, 9728 NX Groningen (NL); ROSEN, Robert, 9718 MX Groningen (NL); STATE, Mihai, 9728 NX Groningen (NL); A. PIERS, Patricia, 9728 NX Groningen (NL)
(74) Representative: Carpmaels & Ransford LLP
(86) International application number: PCT/EP2022/050192
(87) International publication number: WO 2022/148804

(56) References cited:
- EP-A1- 0 941 717
- US-A1- 2017 348 092
- US-A1- 2018 338 827

## Description

### CROSS-REFERENCE TO RELATED APPLICATIONS

This application claims the benefit under 35 U.S.C. § 119(e) of U.S. Provisional Patent Application No. 63/134946, filed January 7, 2021.

### BACKGROUND

Ophthalmic lenses may be utilized to correct optical aberrations of an individual's eye. For example, glasses and contact lenses may be utilized to correct for spherical aberration or astigmatism present in an individual's eye.

Ophthalmic lenses in the form of intraocular lenses may also be utilized to correct optical aberrations of an individual's eye. Intraocular lenses are typically implanted within the capsular bag of an individual's eye and often replace the natural lens present within an individual's eye. The natural lens may have become clouded due to cataracts or may need to be replaced due to other maladies of the individual's eye.

US2018338827A1 describes a composite light adjustable intraocular lens which includes an intraocular lens (IOL), a light adjustable lens, attached to the intraocular lens, and haptics.

The intraocular lens preferably improves the vision of the individual's eye such that additional ophthalmic lenses in the form of glasses or contact lenses may not be needed. However, certain side effects may result from the implantation of the ophthalmic lens. One such side effect that may impact vision is negative dysphotopsia. Negative dysphotopsia may have high prevalence immediately after cataract surgery and may reduce over months and years. Improved methods of determining and reducing negative dysphotopsia is thus desired.

### SUMMARY

Apparatuses, systems, and methods disclosed herein may be directed to reducing negative dysphotopsia in an individual's eye. Such apparatuses, systems, and methods may include determining an angle kappa of an individual's eye. Such apparatuses, systems, and methods may further include tilt adjustable intraocular lenses.

The invention is defined by the appended independent claims. Certain embodiments are defined in the appended dependent claims.

### BRIEF DESCRIPTION OF THE DRAWINGS

Features and advantages of the systems, apparatuses, and methods as disclosed herein will become appreciated as the same become better understood with reference to the specification, claims, and appended drawings wherein:
FIG. 1 illustrates a cross sectional view of a phakic eye including a natural crystalline lens.
FIG. 2 illustrates a cross sectional view of the eye shown in FIG. 1 in which the natural lens has been replaced by an intraocular lens.
FIG. 3 illustrates a top view of an intraocular lens according to an embodiment of the present disclosure.
FIG. 4 illustrates a side cross sectional view of the intraocular lens shown in FIG. 3 along line IV-IV shown in FIG. 3.
FIG. 5 illustrates a top view of an intraocular lens according to an embodiment of the present disclosure.
FIG. 6 illustrates a side cross sectional view of the intraocular lens shown in FIG. 5 along line VI-VI shown in FIG. 5.
FIG. 7 illustrates a side cross sectional view of the intraocular lens shown in FIG. 6 with the optic tilted from the position shown in FIG. 6.
FIG. 8 illustrates a top view of an intraocular lens according to an embodiment of the present disclosure.
FIG. 9 illustrates a side cross sectional view of the intraocular lens shown in FIG. 8 along line IX-IX shown in FIG. 8.
FIG. 10 illustrates a schematic view of a system.

### DETAILED DESCRIPTION

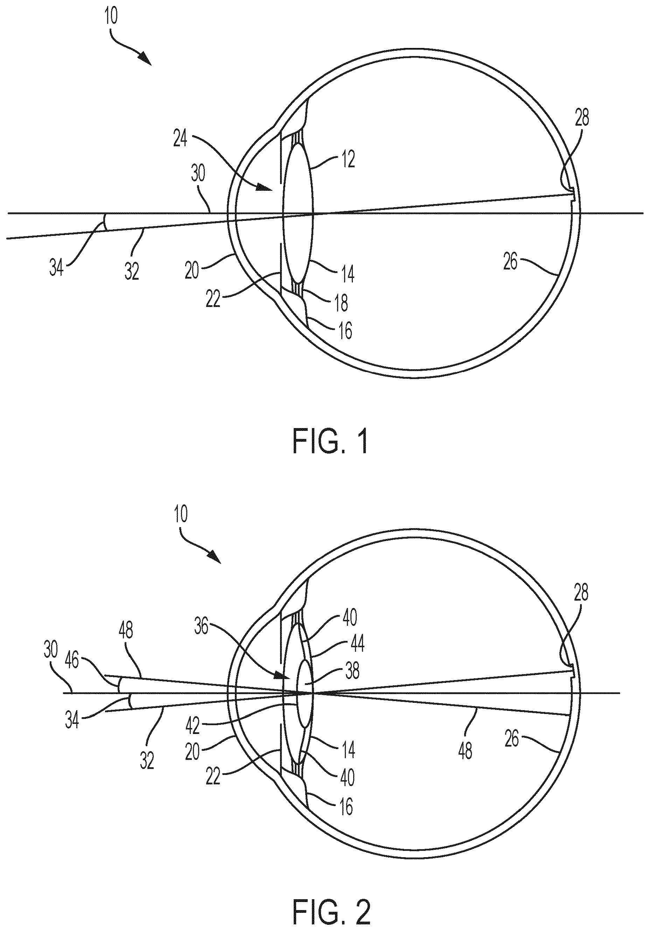

FIG. 1 illustrates a cross sectional view of a phakic eye 10 including a natural crystalline lens 12. The lens 12 may be positioned within a capsular bag 14 (more clearly shown in FIG. 2). The capsular bag 14 may be coupled to a ciliary muscle 16 via zonules 18. The ciliary muscle 16, via the zonules 18, controls the shape and position of the natural lens 12, which allows the eye 10 to focus on distant and near objects. Distant vision is provided when the ciliary muscle 16 pulls the zonules 18, which thus pull the natural lens 12 so that the capsular bag 14 is generally flatter and has a longer focal length (lower optical power). Near vision is provided as the ciliary muscle 16 contracts, thereby relaxing the zonules 18 and allowing the natural lens 12 to return to a more rounded, unstressed state that produces a shorter focal length (higher optical power).

The eye 10 includes a cornea 20 and an iris 22 disposed between the cornea 20 and the natural lens 12. The iris 22 provides a variable pupil 24 that dilates under lower lighting conditions (scotoptic vision) and contracts under brighter lighting conditions (photopic vision).

The eye 10 includes a retina 26 that receives light in the form of an image. The retina 26 includes the fovea 28, which is a small depression in the retina 26 at which visual acuity is the highest.

The eye 10 has a pupillary axis 30, which is a line perpendicular to the cornea 20 that intersects the center of the pupil 24. The eye 10 also has a visual axis 32, which is a line joining the fixation point of the eye 10 to the nodal point of the eye 10. An angle 34 between the pupillary axis 30 and the visual axis 32 is referred to as "angle kappa."

Angle kappa 34 is typically in the nasal direction of an eye 10, as the fovea 28 is typically in the temporal direction of the eye 10. Notably, the magnitude of angle kappa 34 may vary for different individuals' eyes based on the particular physiology of the individual's eye. An average magnitude of angle kappa 34 is about five degrees, with a standard deviation of about 2.5 degrees. However, greater variation may be observed for different individuals. For example, an angle kappa 34 may be about 7.5 degrees or greater (e.g., about ten degrees or greater). An angle kappa 34 may be between about 2.5 degrees and about zero degrees in certain individuals, and may be about zero degrees in certain individuals. The amount of angle kappa may vary greatly in different individuals. The orientation of the angle kappa 34 may vary in different individuals as well.

FIG. 2 illustrates a cross sectional view of the eye 10 in which the natural lens 12 has been replaced by an intraocular lens 36. The natural lens 12 may be replaced by the intraocular lens 36 for a variety of reasons, which may include for example, clouding of the natural lens 12 due to cataracts. Other maladies of the eye may require replacement of the natural lens 12.

The intraocular lens 36 may include an optic 38 and a platform including haptics 40 extending outward from the optic 38. The intraocular lens 36, and the optic 38, may include an anterior surface 42, and a posterior surface 44 facing opposite the anterior surface 42. One or more of the surfaces 42, 44 of the optic 38 may be configured to form an image on the retina 26 of the eye 10. The optic 38 may be configured to improve the vision of the eye such that other ophthalmic lenses (e.g., contact lenses, glasses) may not be needed. One or more of the surfaces 42, 44, for example, may be a refractive surface, a diffractive surface, or a combination of refractive or diffractive surfaces to form the image on the retina 26. In one embodiment, the intraocular lens 36, and the optic 38, may be multifocal, to provide a plurality of focuses. For example, a far focus corresponding to distance vision, and a near focus corresponding to near vision may result. In other embodiments, one or more intermediate focuses between the far focus and the near focus may result. In one embodiment, the intraocular lens 36 may be configured as an extended depth of focus lens, with an extended depth of focus between a far focus and a near focus.

The haptics 40 may be configured to center the optic 38 within the capsular bag 14. The haptics 40 may have a variety of configurations as desired.

The implantation of the intraocular lens 36 may produce negative dysphotopsia in the individual's eye. The negative dysphotopsia may be based on the implantation of the intraocular lens 36 in the individual's eye. According to embodiments herein, it is believed that an indicator of negative dysphotopsia based on the implantation of the intraocular lens 36 in the individual's eye may be the tilt of the optic 38 of the intraocular lens 36 with respect to the visual axis 32. The tilt of the optic 38 of the intraocular lens 36 with respect to the visual axis 32 may be caused by factors.

Such factors may include the angle kappa 34 (the angle between the pupillary axis 30 and the visual axis 32) and the tilt of the optic 38 of the intraocular lens 36 with respect to the pupillary axis 30 (the angle 46 between the pupillary axis 30 and the optical axis 48 of the optic 38).

As such, according to embodiments disclosed herein, the angle kappa 34 may be determined for an individual's eye. The angle kappa 34 of the individual's eye in embodiments may be determined by being measured pre-operatively, prior to the intraocular lens 36 being implanted into the patient's eye. The angle kappa 34 may be determined via a variety of methods. Such methods may include utilizing one or more of Purkinje images or a distance between a center of Placido rings and a center of the cornea 20 of the individual's eye. In embodiments, a method utilizing ultrasound biomicroscopy and corneal topography may be utilized. The methods utilized may involve measurements of the biometry of an individual's eye. A variety of other methods may be utilized as desired. The angle kappa 34 may be determined based on these measurements.

A determination of the likelihood of having negative dysphotopsia after cataract surgery may be produced in the individual's eye. In embodiments, the determination may be produced based on the angle kappa 34 of the individual's eye, and may include a determination of the risk of negative dysphotopsia in the individual's eye based on implantation of an intraocular lens in the individual's eye.

A variety of methods may be utilized to produce the determination of the likelihood of having negative dysphotopsia after cataract surgery in the individual's eye. In embodiments, the determined angle kappa 34 may be compared to a threshold value of angle kappa 34. It may be determined whether the angle kappa 34 meets such a threshold (by equaling or being higher than the threshold) to produce the determination of negative dysphotopsia. For example, if the determined angle kappa 34 is equal to or higher than a threshold value of five degrees, then a determination may be made that negative dysphotopsia in the individual's eye may occur. Other thresholds may be utilized. It is envisioned that the threshold value may be equal to or greater than two, three or four degrees. It is also envisioned that the threshold value may be equal to or greater than six, seven, eight, nine or even 10 degrees.

The threshold value may be determined in a variety of manners. For example, data from patients that have already had intraocular lenses implanted in their eyes may be utilized. Such data may include parameters of the angles of the intraocular lenses and the angle kappas, and whether negative dysphotopsia is experienced by such individuals. As an example, an average tilt of the optic 38 of the intraocular lens 36 with respect to the pupillary axis 30 (the angle 46 between the pupillary axis 30 and the optical axis 48 of the optic 38) may be four degrees. Further, a minimum tilt of the optic 38 of the intraocular lens 36 with respect to the visual axis 32 in individuals experiencing negative dysphotopsia may be eight degrees. As such, a threshold value of angle kappa 34 may be set at five degrees, at which a measured value of angle kappa 34 at or greater than this threshold may result in negative dysphotopsia in the individual's eye based on implantation of the intraocular lens in the individual's eye.

The determination of negative dysphotopsia in the individual's eye accordingly may be based on one or more measurements of angle kappa, which may include a measurement of angle kappa of the individual's eye, and may include measurements of angle kappa of other individuals. Other forms of biometry may be utilized to produce the determination of negative dysphotopsia in the individual's eye. Such forms of biometry may be those used to perform a power calculation for the intraocular lens.

The determination of the likelihood of having negative dysphotopsia after cataract surgery in the individual's eye may be produced in a variety of forms. In embodiments, the determination may comprise a probability of negative dysphotopsia occurring. The probability may be a probability of negative dysphotopsia in the individual's eye based on one or more measurements of angle kappa, and may be based on implantation of the intraocular lens in the individual's eye. For example, upon the angle kappa 34 being determined, a likelihood of negative dysphotopsia may be produced. A clinician accordingly may use such a probability to determine whether there is a high likelihood of negative dysphotopsia occurring following implantation of the intraocular lens. The clinician accordingly may determine whether to proceed with implantation of the intraocular lens based on the likelihood of negative dysphotopsia occurring.

In embodiments, the determination may comprise a binary result of negative dysphotopsia occurring in the individual's eye (e.g., a yes or no determination based on the measured angle kappa 34).

In embodiments, the clinician may determine a type of intraocular lens that may be implanted in the patient's eye based on the likelihood of negative dysphotopsia occurring (e.g., a risk estimation of negative dysphotopsia). A clinician may select an intraocular lens that may be designed to address the presence of negative dysphotopsia, for example a tilt adjustable intraocular lens or a lens having an optical axis that is tilted, or other form of lens as disclosed herein.

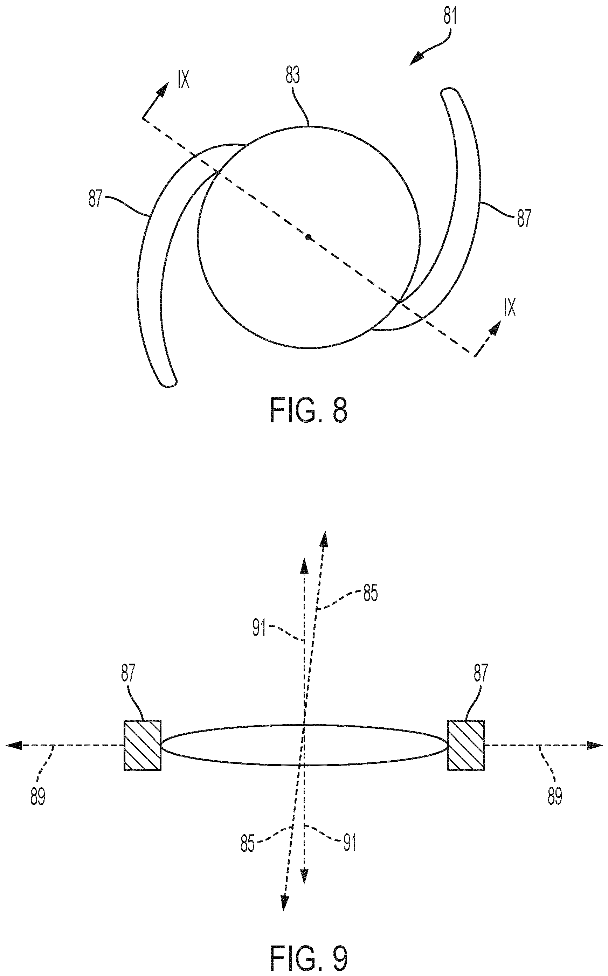

A clinician may select an intraocular lens that may provide angle kappa correction in the individual's eye. The correction may be a whole or partial correction in embodiments. The intraocular lens may have an optic with an optical axis that is tilted with respect to a platform to provide the angle kappa correction in the individual's eye. Such an optic may comprise an optic as shown in FIGS. 8 and 9 for example. The type of intraocular lens may be selected based on a degree of tilt of the optical axis with respect to the platform that provides the angle kappa correction in the individual's eye. For example, if six degrees of angle kappa correction are needed, then the clinician may select an optic with an optical axis the provides the six degrees of angle kappa correction. In embodiments, the intraocular lens may be selected from a plurality of intraocular lenses having a different degree of tilt of an optical axis of an optic with respect to a platform. For example, the intraocular lens having six degrees of angle kappa correction may be selected from a set of intraocular lenses having five degrees, six degrees, and seven degrees of angle kappa correction. The clinician may select the intraocular lens having six degrees of angle kappa correction from this set.

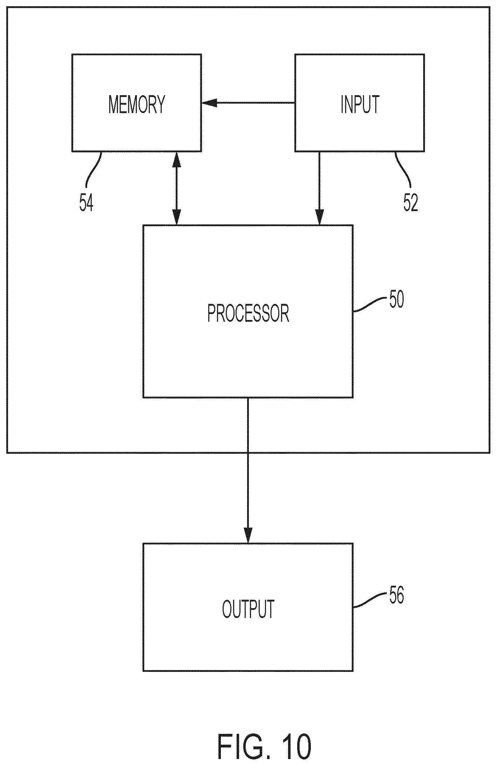

The determination of negative dysphotopsia in the individual's eye based on implantation of the intraocular lens in the individual's eye may be made in a variety of manners. For example, a processor 50 as shown in FIG. 10 may be utilized to make such a determination. The processor 50 may receive an input 52 of the angle kappa 34. The processor 50 may operate an algorithm stored in memory 54 to produce an output 56 of the determination of negative dysphotopsia in the individual's eye based on implantation of the intraocular lens in the individual's eye. In embodiments, a machine learning algorithm may be utilized. The machine learning algorithm may utilize feedback of negative dysphotopsia following implantation of one or more intraocular lenses in one or more individual's eyes to determine if negative dysphotopsia will occur in this particular individual's eye. As such, the processor 50 may rely on data from other procedures, which may include parameters of the angles of the intraocular lenses and the angle kappas, and whether negative dysphotopsia is experienced by such individuals. The data may be collected as a pre-cataract surgery angle kappa, and a post-cataract surgery negative dysphotopsia occurrence, among other forms of data that may be utilized.

In embodiments, a tilt adjustable intraocular lens may be selected and utilized. The tilt adjustable intraocular lens may be utilized to address the determination of negative dysphotopsia in the individual's eye. For example, if there is a high likelihood of negative dysphotopsia determined for an individual's eye, then a tilt adjustable intraocular lens may be selected for implantation in the individual's eye. Such a tilt adjustable intraocular lens may be configured to have the optic centered with respect to the visual axis 32 by adjusting the tilt of the optic, and may be centered with a relatively small variation in the angle from the visual axis.

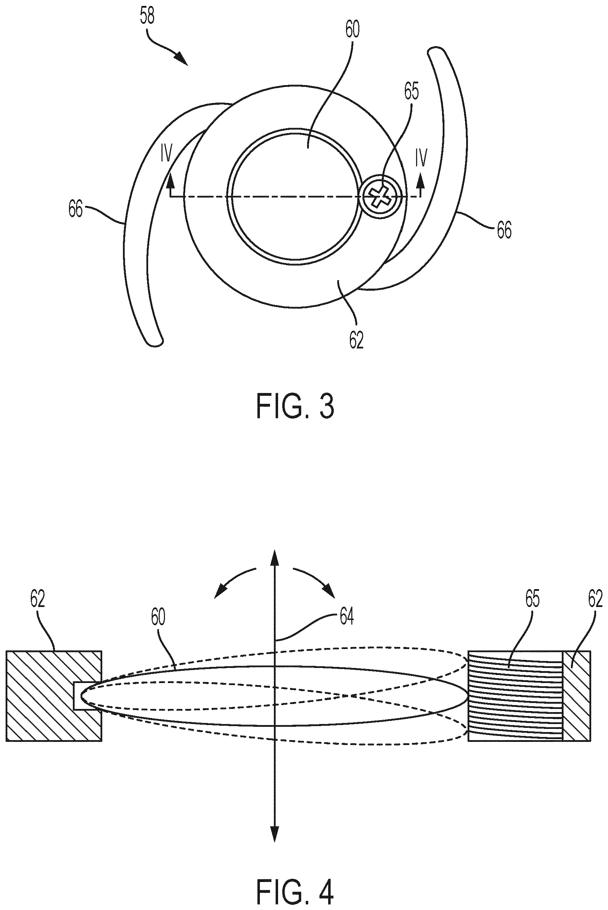

FIG. 3, for example, illustrates an embodiment of a tilt adjustable intraocular lens 58. The lens 58 may include an optic 60 and a platform 62 coupled to the optic 60 and configured to support the optic 60. The optic 60 may have an optical axis 64 (marked in FIG. 4) that may be adjustable with respect to the platform 62. The platform 62 may include haptics 66 extending outward from the optic 60 and configured to support the optic 60. FIG. 3 illustrates a top view of such an embodiment. A tilt of the optical axis 64 may be adjustable with respect to the platform 62.

FIG. 4 illustrate a cross sectional view of the embodiment of FIG. 3 along line IV-IV. The lens 58 may include a screw coupling 65 that may couple the platform 62 to the optic 60 and may allow the optical axis 64 to tilt with respect to the platform 62. For example, as the screw coupling 65 is rotated in one direction, the optical axis 64 may tilt as shown in FIG. 4, and as the screw coupling 65 is rotated in the other direction, the optical axis 64 may tilt in another direction. The amount of tilt of the optical axis 64 may be controlled by the amount that the screw coupling 65 is rotated.

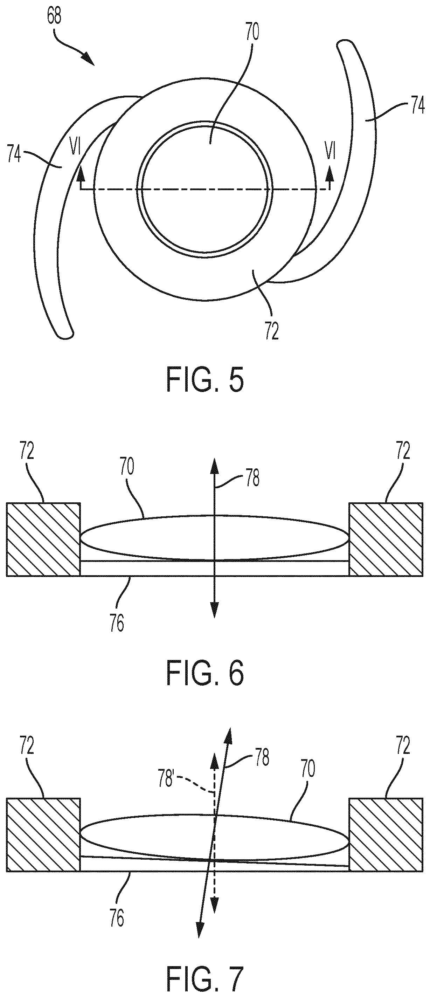

FIG. 5 illustrates an embodiment of a tilt adjustable intraocular lens 68. The lens 68 includes an optic 70 and a platform 72 coupled to the optic 70 and configured to support the optic 70. The platform 72 may include haptics 74 extending outward from the optic 70 and configured to support the optic 70. FIG. 5 illustrates a top view of such an embodiment. A tilt of the optical axis may be adjustable with respect to the platform 72.

FIG. 6 illustrates a cross sectional view of the embodiment of FIG. 5 along line VI-VI. The optic 70 may be positioned upon a portion 76 of the platform 72 that may be configured to be ablated, for example with a laser. The portion 76 may be ablated to allow the optical axis 78 to tilt with respect to the platform 72. FIG. 7, for example, illustrates the portion 76 ablated to vary a shape of the portion 76 to cause the optic 70 to tilt. The optical axis 78 has tilted from the position marked as 78' to the position of the optical axis 78. In embodiments, one or more of the optic 70 or the platform 72 may be ablated to tilt the optical axis 78. For example, one or more surfaces of the optic 70 may be laser ablated according to embodiment herein to provide an angle kappa correction.

Implantation of a tilt adjustable intraocular lens may include determining the visual axis 32 of the individual's eye. The tilt of the optical axis of the intraocular lens may then be adjusted to center the optic with respect to the visual axis 32. The adjustment may occur prior to implantation of the intraocular lens, or during or after implantation. Further, the determination of the visual axis 32 of the individual's eye may occur prior to, during, or after implantation of the intraocular lens. The clinician may iteratively determine the visual axis 32 and adjust the tilt of the optical axis to center the optic with respect to the visual axis 32.

In embodiments, the clinician may provide a tilt adjustment of an optical axis of an optic of an intraocular lens with respect to a platform of the intraocular lens, with the tilt adjustment being provided based on the determination of negative dysphotopsia in the individual's eye based on one or more measurements of angle kappa. The tilt adjustment may be provided as a determination of whether to provide the tilt adjustment, based on the determination of negative dysphotopsia in the individual's eye based on one or more measurements of angle kappa. For example, if a low propensity of negative dysphotopsia is determined, then a clinician may determine that no tilt adjustment is needed. Conversely, if a high propensity of negative dysphotopsia is determined, then a clinician may determine that a tilt adjustment is needed.

In embodiments, the tilt adjustment may be provided as an amount of tilt adjustment based on the determination of negative dysphotopsia in the individual's eye based on one or more measurements of angle kappa. For example, the clinician may be able to determine how much of a tilt adjustment may be needed, based on the determination of negative dysphotopsia in the individual's eye based on one or more measurements of angle kappa. The clinician may determine the degree to which the tilt should be adjusted. Such an adjustment, in embodiments, may be performed post-implantation of the intraocular lens into the individual's eye. For example, a corrective procedure may be performed to provide an angle kappa correction after the individual's eye already has been implanted with the intraocular lens.

The tilt adjustment may provide an angle kappa correction in the individual's eye. The clinician may perform the tilt adjustment intraoperatively in embodiments. One or more of a mechanical adjustment or a laser ablation, as disclosed herein, may be utilized. For example, according to methods herein, a laser ablation may be performed to one or more surfaces of the optic 70 to provide an angle kappa correction. The amount of material to be ablated may be determined based on the desired angle kappa correction. The tilt of the optical axis may be set based on the measured angle kappa of the individual's eye (e.g., the tilt to center with the visual axis).

The amount of tilt may be scaled so that the clinician may control the tilt during implantation within a tilt correction range. The tilt correction range may be based on pre-existent analytical models. The analytical models, in embodiments, may be configured to collect pre and post-surgery biometry (such as intraocular lens tilt, angle kappa, etc.) and produce a prediction model. In embodiments, average values may be utilized.

FIGS. 8 and 9 illustrate an embodiment of an intraocular lens 81 having an optic 83 having an optical axis 85, and a platform 87 coupled to the optic 83 and configured to support the optic 83. The platform 87 may include haptics in embodiments. Such an embodiment may comprise an intraocular lens having a tilted optical axis that is non-adjustable. The angle of the tilted optical axis, for example, may be pre-set.

As shown in FIG. 9, the optical axis 85 may be tilted with the respect to the platform 87. The platform 87, for example, may have an orientation plane 89 that the platform 87 is configured to position the intraocular lens 81 within the eye in. However, the optical axis 85 may be tilted with respect to this plane 89 such that the optical axis 85 is not perpendicular 91 with the plane 89 (as shown in FIG. 9)

The optical axis 85 may be tilted to provide angle kappa correction in an individual's eye. As such, the tilt of the optical axis 85 may be set such that the optical axis 85 is centered with respect to the visual axis 32. In embodiments, the optical axis 85 may be set based on the measured angle kappa of the individual's eye (e.g., the tilt to center with the visual axis).

The optical axis 85, in embodiments, may be pre-set based on an angle kappa of one or more individual's eyes. For example, the angle kappa of many individuals may be known and the optical axis 85 may be pre-set based on an average angle kappa of these many individuals. As such, a clinician may select the intraocular lens 81 having an average value of angle kappa correction. In embodiments, the type of intraocular lens (including the angle of the optical axis) may be selected according to methods disclosed herein, including selection for providing an angle kappa correction in an individual's eye, with the intraocular lens being selected based on a determination of negative dysphotopsia in the individual's eye based on one or more measurements of angle kappa.

Centering the optic with respect to the visual axis 32 may beneficially reduce the prevalence of negative dysphotopsia in the individual's eye. The embodiments disclosed herein may be utilized with one or more of a monofocal optic, a multifocal optic, or an extended depth of focus optic. The optics may be refractive and/or diffractive. Tilt adjustable intraocular lenses may be applied to compensate for the negative impact of large angle kappa in image quality, particularly for multifocal intraocular lenses.

Features of embodiments may be modified, substituted, excluded, or combined as desired.

In addition, the methods herein are not limited to the methods specifically described, and may include methods of utilizing the systems and apparatuses disclosed herein.

In embodiments, a method may include implanting an intraocular lens in an individual's eye. In embodiments, the intraocular lens may include features as disclosed herein, including an optic having an optical axis, and a platform coupled to the optic and configured to support the optic, wherein a tilt of the optical axis is adjustable with respect to the platform.

A method may include determining a visual axis of the individual's eye. The method may include adjusting the tilt of the optical axis with respect to the platform to center the optic with respect to the visual axis of the individual's eye.

In embodiments, a screw coupling may couple the platform to the optic, and the method may further comprise adjusting the screw coupling to adjust the tilt of the optical axis with respect to the platform to center the optic with respect to the visual axis of the individual's eye.

In embodiments, a method may include laser ablating a portion of one or more of the optic or the platform to adjust the tilt of the optical axis with respect to the platform to center the optic with respect to the visual axis of the individual's eye.

A method may include adjusting the tilt of the optical axis prior to implantation of the intraocular lens in the individual's eye. A method may include adjusting the tilt of the optical axis during or after implantation of the intraocular lens in the individual's eye.

In embodiments, the visual axis of the individual's eye may be determined based on an angle kappa of the individual's eye.

A method may include measuring the angle kappa utilizing one or more of Purkinje images or a distance between a center of Placido rings and a center of a cornea of the individual's eye.

In embodiments, a method may include implanting an intraocular lens in an individual's eye. The intraocular lens may include an optic having an optical axis, and a platform coupled to the optic and configured to support the optic. The optical axis may be tilted with respect to the platform to provide angle kappa correction in the individual's eye.

In embodiments, the intraocular lens may be selected based on an angle kappa of the individual's eye. The optical axis may be tilted with respect to the orientation plane of the haptics for the intraocular lens.

In embodiments, a degree of the tilt of the optical axis is set based on an angle kappa of one or more individuals' eyes.

The method may include determining a visual axis of the individual's eye. A tilt of the optical axis with respect to the platform may center the optic with respect to the visual axis of the individual's eye. The visual axis of the individual's eye may be determined based on an angle kappa of the individual's eye. The method may include measuring the angle kappa utilizing one or more of Purkinje images or a distance between a center of Placido rings and a center of a cornea of the individual's eye.

According to embodiments herein, the processor 50 shown in the system of FIG. 10 may comprise a central processing unit (CPU) or other form of processor. In certain embodiments the processor 50 may comprise one or more processors. The processor 50 may include one or more processors that are distributed in certain embodiments, for example, the processor 50 may be positioned remote from other components of the system or may be utilized in a cloud computing environment. The memory 54 may comprise a memory that is readable by the processor 50. The memory 54 may store instructions, or features of intraocular lenses, or other parameters that may be utilized by the processor 50 to perform the methods disclosed herein. The memory 54 may comprise a hard disk, read-only memory (ROM), random access memory (RAM) or other form of non-transient medium for storing data. The input 52 may comprise a port, terminal, physical input device, or other form of input. The port or terminal may comprise a physical port or terminal or an electronic port or terminal. The port may comprise a wired or wireless communication device in certain embodiments. The physical input device may comprise a keyboard, touchscreen, keypad, pointer device, or other form of physical input device. The input 52 may be configured to provide an input to the processor 50. The output 56 may comprise a variety of forms of output, including non-transitory digital signals, a computer screen output, a printed output, or other forms of output as desired. Other configurations of systems may be utilized in embodiments.

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings herein without departing from the present specification. Lastly, the terminology used herein is for the purpose of describing particular embodiments only, and is not intended to limit the scope of systems, apparatuses, and methods as disclosed herein, which is defined solely by the claims. Accordingly, the systems, apparatuses, and methods are not limited to that precisely as shown and described.

Certain embodiments of systems, apparatuses, and methods are described herein, including the best mode known to the inventors for carrying out the same. Of course, variations on these described embodiments will become apparent to those of ordinary skill in the art upon reading the foregoing description. The inventor expects skilled artisans to employ such variations as appropriate, and the inventors intend for the systems, apparatuses, and methods to be practiced otherwise than specifically described herein. Accordingly, the systems, apparatuses, and methods include all modifications and equivalents of the subject matter recited in the claims appended hereto as permitted by applicable law. Moreover, any combination of the above-described embodiments in all possible variations thereof is encompassed by the systems, apparatuses, and methods unless otherwise indicated herein or otherwise clearly contradicted by context.

Groupings of alternative embodiments, elements, or steps of the systems, apparatuses, and methods are not to be construed as limitations. Each group member may be referred to and claimed individually or in any combination with other group members disclosed herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

The terms "a," "an," "the" and similar referents used in the context of describing the systems, apparatuses, and methods (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the systems, apparatuses, and methods and does not pose a limitation on the scope of the systems, apparatuses, and methods otherwise claimed. No language in the present specification should be construed as indicating any non-claimed element essential to the practice of the systems, apparatuses, and methods.

## Claims

1. An intraocular lens (58) for reducing negative dysphotopsia comprising:
an optic (60) having an optical axis (64); and
a platform (62) coupled to the optic and configured to support the optic,
wherein a tilt of the optical axis is adjustable with respect to the platform, wherein a screw coupling (65) couples the platform to the optic and allows the optical axis of the optic to tilt with respect to the platform.

2. An intraocular lens (68) for reducing negative dysphotopsia comprising:
an optic (70) having an optical axis (78); and
a platform (72) coupled to the optic and configured to support the optic,
wherein a tilt of the optical axis is adjustable with respect to the platform, wherein a portion (76) of one or more of the optic or the platform is configured to be laser ablated to allow the optical axis of the optic to tilt with respect to the platform.

3. An intraocular lens (81) for reducing negative dysphotopsia comprising:
an optic (83) having an optical axis (85); and
a platform (87) coupled to the optic and configured to support the optic,
wherein the optical axis is tilted with respect to the platform to provide angle kappa correction in an individual's eye, wherein the angle of the tilted optical axis is pre-set.

4. The intraocular lens of claim 3, wherein the optical axis is tilted with respect to an orientation plane (89) of haptics coupled to the intraocular lens.

5. The intraocular lens of claim 3, wherein a degree of tilt of the optical axis is set based on an angle kappa of one or more individuals' eyes.

6. A method for reducing negative dysphotopsia comprising:
measuring an angle kappa of an individual's eye prior to an implantation of an intraocular lens in the individual's eye; and
producing, using a processor, a determination, based on the angle kappa of the individual's eye, of the risk of negative dysphotopsia in the individual's eye following cataract surgery based on implantation of an intraocular lens in the individual's eye.

7. The method of claim 6, wherein the determination includes a probability of negative dysphotopsia in the individual's eye based on implantation of the intraocular lens in the individual's eye.

8. The method of claim 6, wherein the determination is produced based on whether the angle kappa meets a threshold value.

9. The method of claim 6, wherein the threshold value is higher than or equal to five degrees.

10. The method of claim 6, wherein the threshold value is higher than or equal to five degrees.

11. The method of claim 6, wherein the threshold value is higher than or equal to five degrees.

12. The method of claim 6, wherein the angle kappa is measured utilizing one or more of Purkinje images or a distance between a center of Placido rings and a center of a cornea of the individual's eye.

13. The method of claim 6, wherein the determination is produced based on a machine learning algorithm utilizing feedback of negative dysphotopsia following implantation of one or more intraocular lenses in one or more individuals' eyes.

14. The method of claim 13, further comprising selecting a tilt adjustable intraocular lens to implant in the individual's eye based on the determination of negative dysphotopsia in the individual's eye.

15. The method of claim 6, further comprising selecting an intraocular lens to implant in the individual's eye based on the determination of negative dysphotopsia in the individual's eye, wherein the intraocular lens has a pre-set tilted optical axis to provide angle kappa correction in the individual's eye.

## Patentansprüche

1. Intraokularlinse (58) zum Verringern von negativer Dysphotopsie, umfassend:
ein optisches Element (60), das eine optische Achse (64) aufweist; und
eine Plattform (62), die mit dem optischen Element gekoppelt und konfiguriert ist, um das optische Element zu tragen,
wobei eine Neigung der optischen Achse in Bezug auf die Plattform anpassbar ist, wobei eine Schraubkopplung (65) die Plattform mit dem optischen Element koppelt und ermöglicht, dass sich die optische Achse des optischen Elements in Bezug auf die Plattform neigt.

2. Intraokularlinse (68) zum Verringern von negativer Dysphotopsie, umfassend:
ein optisches Element (70), das eine optische Achse (78) aufweist; und
eine Plattform (72), die mit dem optischen Element gekoppelt und konfiguriert ist, um das optische Element zu tragen,
wobei eine Neigung der optischen Achse in Bezug auf die Plattform anpassbar ist, wobei ein Abschnitt (76) eines oder mehrerer des optischen Elements oder der Plattform konfiguriert ist, um mit Laser abgetragen zu werden, um zu ermöglichen, dass sich die optische Achse des optischen Elements in Bezug auf die Plattform neigt.

3. Intraokularlinse (81) zum Verringern von negativer Dysphotopsie, umfassend:
ein optisches Element (83), das eine optische Achse (85) aufweist; und
eine Plattform (87), die mit dem optischen Element gekoppelt und konfiguriert ist, um das optische Element zu tragen,
wobei die optische Achse in Bezug auf die Plattform geneigt ist, um eine Korrektur von Winkel Kappa in einem Auge einer Person bereitzustellen, wobei der Winkel der geneigten optischen Achse voreingestellt ist.

4. Intraokularlinse nach Anspruch 3, wobei die optische Achse in Bezug auf eine Ausrichtungsebene (89) von Haptik geneigt ist, die mit der Intraokularlinse gekoppelt ist.

5. Intraokularlinse nach Anspruch 3, wobei der Neigungsgrad der optischen Achse basierend auf einem Winkel Kappa eines oder mehrerer Augen der Personen eingestellt wird.

6. Verfahren zum Verringern von negativer Dysphotopsie, umfassend:
Messen eines Winkels Kappa eines Auges der Person vor einer Implantation einer Intraokularlinse in das Auge der Person; und
Erstellen, unter Verwendung eines Prozessors, einer Bestimmung, basierend auf dem Winkel Kappa des Auges der Person, des Risikos von negativer Dysphotopsie in dem Auge der Person nach einer Kataraktoperation basierend auf der Implantation einer Intraokularlinse in das Auge der Person.

7. Verfahren nach Anspruch 6, wobei die Bestimmung eine Wahrscheinlichkeit von negativer Dysphotopsie in dem Auge der Person basierend auf der Implantation der Intraokularlinse in das Auge der Person umfasst.

8. Verfahren nach Anspruch 6, wobei die Bestimmung basierend darauf erstellt wird, ob der Winkel Kappa einen Schwellenwert erreicht.

9. Verfahren nach Anspruch 6, wobei der Schwellenwert größer als oder gleich fünf Grad ist.

10. Verfahren nach Anspruch 6, wobei der Schwellenwert größer als oder gleich fünf Grad ist.

11. Verfahren nach Anspruch 6, wobei der Schwellenwert größer als oder gleich fünf Grad ist.

12. Verfahren nach Anspruch 6, wobei der Winkel Kappa unter Nutzung eines oder mehrerer von Purkinje-Bildern oder eines Abstands zwischen einem Mittelpunkt von Placido-Ringen und einem Mittelpunkt einer Hornhaut des Auges der Person gemessen wird.

13. Verfahren nach Anspruch 6, wobei die Bestimmung basierend auf einem maschinellen Lernalgorithmus erstellt wird, der eine Rückmeldung von negativer Dysphotopsie nach der Implantation einer oder mehrerer Intraokularlinsen in ein oder mehrere Augen der Person nutzt.

14. Verfahren nach Anspruch 13, ferner umfassend ein Auswählen einer neigungsanpassbaren Intraokularlinse, die in das Auge der Person implantiert werden soll, basierend auf der Bestimmung von negativer Dysphotopsie in dem Auge der Person.

15. Verfahren nach Anspruch 6, ferner umfassend das Auswählen einer Intraokularlinse, die in das Auge der Person implantiert werden soll, basierend auf der Bestimmung einer negativen Dysphotopsie in dem Auge der Person, wobei die Intraokularlinse eine voreingestellte geneigte optische Achse aufweist, um die Korrektur von Winkel Kappa in dem Auge der Person bereitzustellen.

## Revendications

1. Lentille intraoculaire (58) permettant de réduire une dysphotopsie négative comprenant :
une optique (60) ayant un axe optique (64) ; et
une plate-forme (62) accouplée à l'optique et conçue pour supporter l'optique,
dans laquelle une inclinaison de l'axe optique est ajustable par rapport à la plate-forme, dans laquelle un accouplement par vis (65) accouple la plate-forme à l'optique et permet à l'axe optique de l'optique de s'incliner par rapport à la plate-forme.

2. Lentille intraoculaire (68) permettant de réduire une dysphotopsie négative comprenant :
une optique (70) ayant un axe optique (78) ; et
une plate-forme (72) accouplée à l'optique et conçue pour supporter l'optique,
dans laquelle une inclinaison de l'axe optique est ajustable par rapport à la plate-forme, dans laquelle une partie (76) d'une ou plusieurs parmi l'optique ou la plate-forme est conçue pour subir une ablation par laser pour permettre à l'axe optique de l'optique de s'incliner par rapport à la plate-forme.

3. Lentille intraoculaire (81) permettant de réduire une dysphotopsie négative comprenant :
une optique (83) ayant un axe optique (85) ; et
une plate-forme (87) accouplée à l'optique et conçue pour supporter l'optique,
dans laquelle l'axe optique est incliné par rapport à la plate-forme pour fournir une correction d'angle kappa dans un œil d'un sujet, dans laquelle l'angle de l'axe optique incliné est préréglé.

4. Lentille intraoculaire selon la revendication 3, dans laquelle l'axe optique est incliné par rapport à un plan d'orientation (89) d'éléments haptiques accouplés à la lentille intraoculaire.

5. Lentille intraoculaire selon la revendication 3, dans laquelle un degré d'inclinaison de l'axe optique est réglé en fonction d'un angle kappa d'un ou plusieurs yeux de sujets.

6. Procédé permettant de réduire une dysphotopsie négative comprenant :
la mesure d'un angle kappa d'un œil d'un sujet avant une implantation d'une lentille intraoculaire dans l'œil du sujet ; et
la production, à l'aide d'un processeur, d'une détermination, en fonction de l'angle kappa de l'œil du sujet, du risque de dysphotopsie négative dans l'œil du sujet à la suite d'une intervention chirurgicale de la cataracte en fonction de l'implantation d'une lentille intraoculaire dans l'œil du sujet.

7. Procédé selon la revendication 6, dans lequel la détermination comporte une probabilité de dysphotopsie négative dans l'œil du sujet en fonction de l'implantation de la lentille intraoculaire dans l'œil du sujet.

8. Procédé selon la revendication 6, dans lequel la détermination est produite en fonction du fait que l'angle kappa respecte une valeur seuil.

9. Procédé selon la revendication 6, dans lequel la valeur seuil est supérieure ou égale à cinq degrés.

10. Procédé selon la revendication 6, dans lequel la valeur seuil est supérieure ou égale à cinq degrés.

11. Procédé selon la revendication 6, dans lequel la valeur seuil est supérieure ou égale à cinq degrés.

12. Procédé selon la revendication 6, dans lequel l'angle kappa est mesuré en utilisant une ou plusieurs parmi des images de Purkinje ou une distance entre un centre de disques de Placido et un centre d'une cornée de l'œil du sujet.

13. Procédé selon la revendication 6, dans lequel la détermination est produite en fonction d'un algorithme d'apprentissage automatique utilisant une rétroaction de dysphotopsie négative à la suite d'une implantation d'une ou plusieurs lentilles intraoculaires dans un ou plusieurs yeux d'un sujet.

14. Procédé selon la revendication 13, comprenant en outre la sélection d'une lentille intraoculaire ajustable en inclinaison à implanter dans l'œil du sujet en fonction de la détermination de dysphotopsie négative dans l'œil du sujet.

15. Procédé selon la revendication 6, comprenant en outre la sélection d'une lentille intraoculaire à implanter dans l'œil du sujet en fonction de la détermination de dysphotopsie négative dans l'œil du sujet, dans lequel la lentille intraoculaire a un axe optique incliné préréglé pour fournir une correction d'angle kappa dans l'œil du sujet.
